(19) Europäisches Patentamt
European Patent Office
Office européen des brevets

(11) Publication number: 0 459 059 B1

(12) **EUROPEAN PATENT SPECIFICATION**

(45) Date of publication of patent specification: 27.07.94

(51) Int. Cl.⁵: **C09J 7/02**, C08L 23/12, C09D 123/12

(21) Application number: 90313875.8

(22) Date of filing: 19.12.90

(54) **Tearable, continuous film medical PSA tape.**

(30) Priority: 03.05.90 US 489092

(43) Date of publication of application:
04.12.91 Bulletin 91/49

(45) Publication of the grant of the patent:
27.07.94 Bulletin 94/30

(84) Designated Contracting States:
DE GB IT

(56) References cited:
EP-A- 0 246 009
EP-A- 0 318 183

(73) Proprietor: MINNESOTA MINING AND MANU-
FACTURING COMPANY
3M Center,
P.O. Box 33427
St. Paul, Minnesota 55133-3427(US)

(72) Inventor: Olsen, Frederick O., c/o Minnesota
Mining and
Manufac. Company,
2501 Hudson Road,
P.O. Box 33427
St. Paul, Minnesota 55133-3427(US)
Inventor: Riedel, John E., c/o Minnesota Min-
ing and
Manufac. Company,
2501 Hudson Road,
P.O. Box 33427
St. Paul, Minnesota 55133-3427(US)
Inventor: Panza, Victor F., c/o Minnesota Min-
ing and
Manufac. Company,
2501 Hudson Road,
P.O. Box 33427
St. Paul, Minnesota 55133-3427(US)

(74) Representative: Baillie, Iain Cameron et al
c/o Ladas & Parry
Altheimer Eck 2
D-80331 München (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a normally tacky, pressure-sensitive adhesive tape having a polyolefin based backing. The tape is particularly useful for medical applications.

Pressure-sensitive adhesive tapes have long been used in the medical arts, in applications such as holding down bandages, I.V. tubes and the like. One of the desired properties of tapes used in these applications is a soft, conformable hand. It is also desirable that the tape be capable of bi-directional tear so that it is easily separated from the roll and is versatile in use. The tape should also have a high moisture vapor permeability and a liquid water impermeability, so that it can provide some protection against water from external sources while allowing perspiration to escape from under the tape.

U.S. Patent No. 4,808,474 to Sipinen discloses a pressure-sensitive adhesive tape with improved toughness that comprises a film that is a blend of crystalline isotactic polypropylene and a compatible flexible polymer, and a coating of pressure-sensitive adhesive on one side of the film. The compatible flexible polymer is selected from ethylene based flexible polymers including, for example, ethylene-vinyl acetate at column 3, lines 35 and 36. Examples 26-28 at column 9 are films that are a blend of the isotactic polypropylene and ethyl vinyl acetate copolymers. The additional incorporation of pigments, fragrances, fillers, and/or dyes is disclosed at column 5, lines 17-23. This patent does not teach or disclose the additional incorporation of materials that will enhance the moisture vapor transmission of the ultimate tape.

Medical tapes having the desired hand and moisture vapor transmission properties are difficult to prepare from polymeric materials because of the problems encountered in adding components to provide a film with specific additional properties. Such additives will typically result in deleterious effects on the integrity and tearability properties of the film. In the case of crystalline polypropylene, the addition of materials to improve processability will typically adversely affect the tearability of the backing. Incorporation of diverse materials such as fillers in significant amounts will result in a compromise in integrity of the film. Such a film will have flaws and usually will have holes, which is unacceptable as a commercial product.

A tape made from polypropylene alone, or even from polypropylene with polyethylene/ethyl vinyl acetate, has almost no moisture vapor transmission properties. When these tapes are applied to the skin over the long term, they pool moisture, which will result eventually in the formation of blisters. None of the above disclosed tapes satisfies the property requirements of a soft, conformable, easily tearable, liquid water impermeable and moisture vapor permeable tape.

### Summary of the Invention

The present invention relates to adhesive composites, and specifically to medical tapes, comprising a film backing and a layer of normally tacky, pressure-sensitive adhesive. The film backing is prepared from a resin comprising

a) about 65 to 98 percent of isotactic polypropylene,

b) about 1 to 20 percent of a polyethylene/ethyl vinyl acetate copolymer comprising about 5 to 40 percent ethyl vinyl acetate, and

c) about 1 to 15 percent of a moisture vapor transmission rate enhancing material.

The backing has an embossed pattern on at least one side that provides for varied thicknesses of the backing such that the backing has an overall thickness of about 0.075 to 0.115 mm. with regions of the backing having a thickness of less than one-half the overall thickness of the backing.

The film backing of the adhesive composite exhibits surprisingly high moisture vapor transmission rates when the backing is exposed to an organic solvent. The adhesive composite exhibits significantly higher moisture vapor transmission rates when the backing is coated with a solvent-based adhesive.

### Brief Description of the Drawing

Fig. 1 depicts a roll of tape made in accordance with the invention;

Fig. 2 is an enlarged stylized and somewhat over simplified perspective view of a portion of the tape shown in Fig. 1, showing the nature of the ridges and valleys in the surface;

Fig. 3 is similar to Fig. 2, showing the effect of applying a stretching force in a longitudinal direction of the tape sufficient to cause the tape to yield along the area at the bottom of the valleys.

Fig. 4 is a top view of a hexagonal embossed pattern that may be imparted to the film.

Fig. 5 is a cross-sectional view of the embossed pattern shown in Fig. 4 taken along line 5-5.

Fig. 6 is a top view of an alternative embossed pattern imparted to the film.

Fig. 7 is a cross-sectional view of the embossed pattern shown in Fig. 6 taken along line 7-7.

Detailed Description of the Drawing

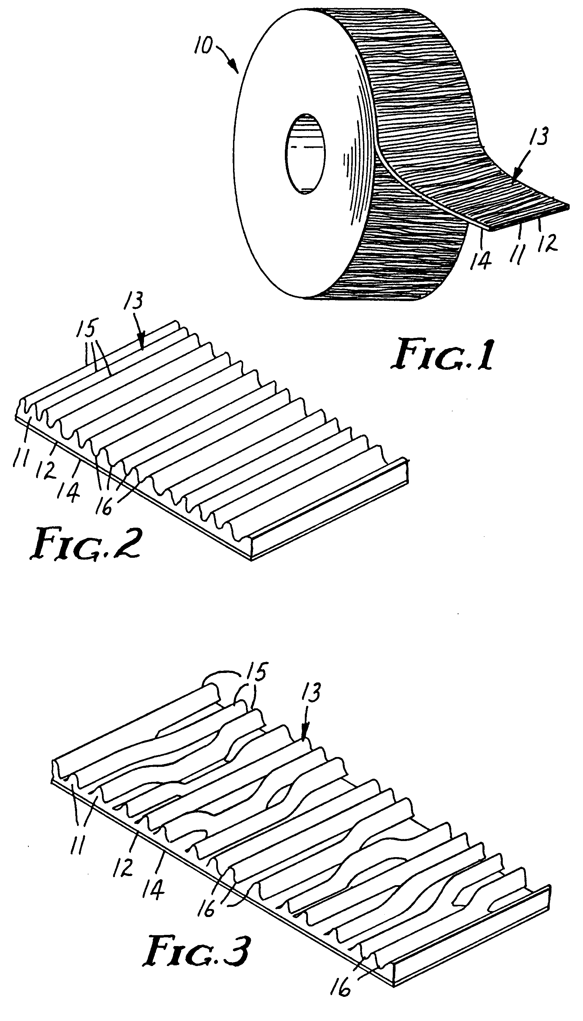

In the drawing, Fig. 1 illustrates a convolutely wound roll **10** of the normally tacky, pressure-sensitive adhesive tape having crystalline polymeric isotactic polypropylene film backing **11**, with smooth face **12** and textured face **13**. A layer of normally tacky, pressure-sensitive adhesive **14** is coated over and firmly adherently bonded to smooth face **12**.

In Fig. 2, which is an enlarged perspective view of a portion of the tape of Fig. 1, textured face **13** is seen to be made up of cord-like ridges **15** separated by valleys **16**, the crest of adjacent ridges **15** being about 250-1500 micrometers (preferably about 500-1000 micrometers) apart. Not all of the valleys have the same thickness, but they average about 50-70% (preferably about 65%) of the calipered film thickness. In measuring the caliper of film **11**, it is practical to use a conventional thickness gauge of the type in which opposed feet respectively contact smooth face **12** and textured face **13**, the foot contacting the latter surface being sufficiently broad to span several ridges **15**. The preferred method of measuring thickness is according to ASTM D-1000, using a 6.45 $cm^2$ foot and 500g weight. This embodiment of the invention closely visually resembles the product of that of U.S. Patent No. 4,237,889 but differs significantly in its physical properties. The product of U.S. Patent No. 4,237,889 employs a backing described as a "substantially untensilized, tough, ductile foil of isotactic polypropylene or linear high density polyethylene," the physical properties resulting "from the quick quenching" (cf. Col. 2, line 37-47), thereby obtaining a film that is not predominantly crystalline. In contrast, the product of the present invention is predominantly crystalline, a characteristic resulting from a comparatively slow quenching of the film during its formation.

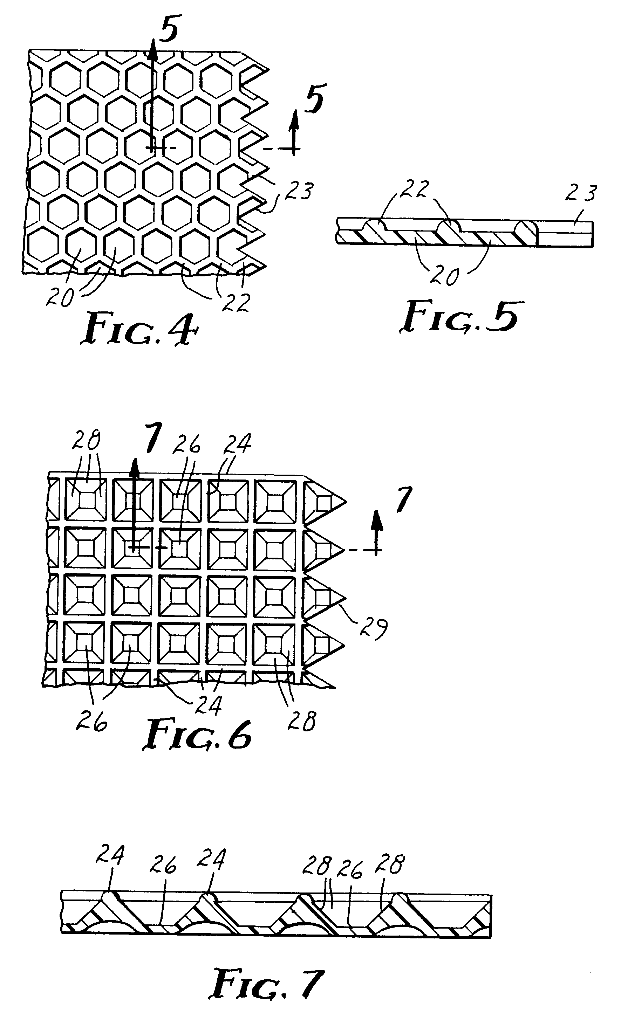

Fig. 4 shows a top view of an embossed pattern that may be imparted to the film of the present invention. The pattern is a hexagonal pattern with hexagons of thinner areas **20** being defined by thick areas **22**. Edge **23** is pinked to provide easier initial tearability and greater comfort to the user.

Fig. 5 is a cross-sectional view of the embossed pattern shown in Fig. 4 taken along line 5-5, with hexagons of thinner areas **20** being defined by thick areas **22**.

Fig. 6 is a top view of an alternative embossed pattern which is a mesh-like pattern of interconnecting ridges **24** defining square thin areas **26**. Areas of gradiated thickness **28** connect ridges **24** with thin areas **26**. Edge **29** is pinked to provide easier initial tearability and greater comfort to the user.

Fig. 7 is a cross-sectional view of the embossed pattern shown in Fig. 6 taken along line 7-7, with ridges **24** being regularly spaced between thin areas **26**. Areas of gradiated thickness **28** connect ridges **24** with thin areas **26**.

Description of the Presently Preferred Embodiments

It has surprisingly been discovered that a polypropylene resin additionally comprising polyethyene/ethyl vinyl acetate polymer in the disclosed ratios will accomodate the incorporation of moisture vapor transmission enhancing materials to provide a tearable continuous film backing. Without the polyethyene/ethyl vinyl acetate polymer, polypropylene resin will not accomodate the incorporation of moisture vapor transmission enhancing materials and be capable of forming a continuous film having a nominal thickness of less than 0.2 mm. with a significant area having a thickness of less than 0.06 mm. The incorporation of moisture vapor transmission enhancing materials in the adhesive composites of the present invention provides important moisture vapor transmission properties to make this polymeric backing tape acceptable for medical uses.

It has also surprisingly been discovered that when the backings of the present invention are intimately contacted with organic solvent, the observed moisture vapor transmission rate actually increases. When the backings of the present invention are coated with a solvent-based adhesive, the observed moisture vapor transmission rate dramatically increases. While not being bound by any theory, it is believed that the intimate contact of the backing with solvent, either alone or in the adhesive coating process, results in some modification in the backing that provides a microfine porous lattice structure in the backing. More contact with organic solvent appears to result in higher observed moisture vapor transmission properties. Thus in the case of solvent-cast adhesive coated backings, the contact of the backing with solvent in the coating step combined with higher heat (about 105-115° C) for drying may drive the solvent into and through the backing to create a microfine porous structure having higher moisture vapor transmission properties.

The properties of the backing are further enhanced by providing the film with an embossed pattern that has a specified amount of thin regions of backing. This pattern may be random in nature, but a pattern that will provide bidirectional tear is most preferred for ease of removal from the roll and for versatility in use in allowing narrow strips to be torn from a wider strip. Most preferably, the pattern will afford prescribed geometrical shaped thicker portions of the backing, such as hexagons or truncated pyramids, that are pleasing to the eye and provide for regular tear characteristics. The pattern will also enhance suppleness and ductility of the tape, assisting in providing a more cloth-like "hand" of the tape. Finally, the pattern can significantly enhance moisture vapor transmission properties of the tape by providing areas of the backing that are relatively thin and therefore much more moisture vapor permeable than uniformly thick backings. In order to provide sufficient thin areas to achieve the desired properties, the pattern is preferably selected such that about 20 percent of the total film backing area has a thickness of no more than about 0.05 mm. More preferably, about 20 percent of the total film backing area has a thickness of no more than about 0.03 mm. Preferably, the thin regions of the film backing comprises no less than 10 percent of the total film backing area.

The tape backing of the present invention is preferably prepared by first mixing the moisture vapor transmission rate enhancing material with the polyethylene/ethyl vinyl acetate blend, together with a small amount of one or more processing adjuvants, such as ethylene glycol. This mixture is then blended with the polypropylene to formulate a master batch of resin that is then processed into polymeric beads to be used in a film extruder. Thorough and complete dispersion of all components in this resin is important to the successful preparation of a continuous film backing.

The film resin is then extruded through a slot extrusion die and, while the extruded film is still molten, passed into a nip between a smooth-surfaced silicone rubber-covered support roll and a temperature controlled metal chill roll having an engraved pattern that is to be imparted to the film backing. The chill roll is maintained at a temperature high enough to ensure that the film will cool slowly enough to attain a predominantly crystalline character. The resulting film has one smooth surface and one "rough" surface. A layer of normally tacky, pressure-sensitive adhesive is applied to the smooth side of the film, and the composite is cut into predetermined lengths or is wound convolutely upon itself about a core to form a roll. The tape thus prepared is slit into rolls, and preferably has pinked edges to further enhance tearability and comfort to the user.

The molecular weights of the isotactic polypropylene resin used can have a wide range, preferably in the range of 200,000 to 400,000. Especially preferred resins have a melt index of from 8 to 12 grams per 10 minutes. Such polypropylene resins are readily available from commercial sources such as Fina Oil and Chemical Company, Deer Park, Texas; Exxon Chemical Americas, Houston, Texas; Himont USA Incorporated, Wilmington, Delaware; and Shell Chemical Company, Houston, Texas. The backing of the adhesive composite of the present invention comprises about 65 to 95 percent by weight of isotactic polypropylene, and preferably about 78 to 86 percent.

The polyethylene/ethyl vinyl acetate copolymer used comprises about 5 to 40 percent by weight of the ethyl vinyl acetate monomer, with the balance being polyethylene. More preferably, the copolymer comprises about 25 to 35 percent by weight of the ethyl vinyl acetate monomer. This copolymer preferably has a density of about 0.92 to 0.96 g/cm$^3$ and a melt index of about 0.2 to 50 grams per 10 minutes, and more preferably about 0.2 to 25.

The moisture vapor transmission rate enhancing material is selected from materials that can afford a physical lattice structure to allow moisture vapor to pass through the film, such as glass beads, silica powder or other finely divided materials that are not readily wetted by polypropylene; diverse resins, such as polyvinyl alcohol, poly(N-vinyl pyrrolidone) or acrylic acid homopolymer; and hydrophilic materials that tend to wick moisture through the film, such as cellulosic fibers or other hydrophyllic fiber-forming materials. The moisture vapor transmission rate enhancing material is present in an amount effective to increase the moisture vapor transmission rate of the film in comparison to films having the same resin content absent the moisture vapor transmission rate enhancing material. Typically, the moisture vapor transmission rate enhancing material is present as about 1 to 15 percent by weight of the film. Preferably, the moisture vapor transmission rate enhancing material is present as about 8 to 12 percent by weight of the film.

Minor amounts of additional components conventional in the tape backing art may also be incorporated in the resin of the film backing. Examples of such materials are processing aids, such as ethylene glycol, pigments, such as titanium dioxide, antistatic agents, light stabilizers, lubricants, and the like.

The pressure-sensitive adhesives that may be used in the present composites are conventional in the art. Such adhesives include acrylate adhesives described in U.S. Pat. No. RE24,906 (Ulrich) or U.S. Pat. No. 4,732,808 (Krampe, et. al.) and phenolic cured rubber based adhesives described in U.S. Pat. No. 2,708,192 (Joesting et. al.). Particularly preferred adhesives are solvent-based to provide enhanced moisture vapor transmission of the overall composite as discussed above. The film backing may optionally be coated with a water-based adhesive, which will not enhance the observed moisture vapor transmission properties of the composite. Prior to coating with a water-based adhesive, the film backing may also be treated with an organic solvent such as ethyl acetate or heptane. This treatment will surprisingly enhance the observed moisture vapor transmission properties of the composite, though not as much as using the solvent-based

4

adhesive.

Tensile and Elongation Testing

Tensile and elongation data were obtained according to procedures described in ASTM Standard Procedures D-822 and D-3759.

Handleometer

Handleometer data were obtained according to the procedure described in INDA Standard Procedure 90.0-75 (R-82).

Moisture Vapor Transmission

Moisture vapor transmission (MVT) data was obtained according to ASTM Standard Procedure E96-80 using the upright cup method.

Comparative Examples 1-4

An extrusion composition was prepared by dry blending together the indicated parts of isotactic polypropylene (PP) resin (L-3576 available from Fina Corp., Dallas, TX) and a polyethylene/ethyl vinyl acetate (PE/EVA) master batch. The master batch was prepared by compounding 2.5% by weight of a $TiO_2$ pigment/polypropylene blend (50% by weight $TiO_2$, #CBE 15100 P White, available from C. B. Edwards, Minneapolis, MN) with the PE/EVA copolymer (#64500, 28/72 PE/EVA, available from Quantum Chemical Corp., Cincinnati, OH) and 1% by weight ethylene glycol. A film ribbon was prepared by extruding the polymer blend through a slot extrusion die (0.25 cm slot thickness) at a temperature of about 230 - 240°C into a nip between a silicone rubber-covered roll and a temperature controlled metal chill roll having an engraved pattern of random ridges and valleys extending circumferencially round the metal chill roll with an average peak to valley dimension of about 0.25 - 0.30 cm. The temperature of the chill roll was maintained at about 65°C, a pressure of about 4.8 - 5.2 bars (70-75 psi) maintained between the casting roll and the squeeze roll, and contact between the film and the chill roll maintained for about six seconds. The overall thickness of the extruded film was a nominal 0.13 mm (5 mils). The resulting film had a one smooth surface and one "rough" surface and was characterized by its tensile properties, hand (handleometer values), and force required to elongate the film (See Table I). All film samples exhibited good machine direction and cross-direction tear properties. The moisture vapor transmission rate for all of the films of Examples 1-4 was less than about 20 $g/m^2/24$ hrs.

### TABLE I

| Example | 1 | 2 | 3 | 4(Control) |
|---|---|---|---|---|
| PP(pph) | 90 | 85 | 80 | 100 |
| PE/EVA(pph) | 10 | 15 | 20 | 0 |
| MD Tensile (kg/cm width) | 2.32 | 2.34 | 2.36 | 2.50 |
| CD Tensile (kg/cm width) | 1.77 | 1.86 | 1.80 | 1.96 |
| Handleometer (grams total) | 149 | 185 | 186 | 182 |
| Force (2% Elong.) (kg/cm width) | 0.36 | 0.88 | 0.80 | 1.39 |
| Force (5% Elong.) (kg/cm width) | 1.89 | 2.10 | 2.11 | 2.50 |
| Force (10% Elong.) (kg/cm width) | 2.27 | 2.32 | 2.30 | – |

Films from examples 1-3 were coated with an acrylic based pressure-sensitive adhesive similar to that described in U.S. Pat. No. RE24,906 (Ulrich) that comprised a 91/9 copolymer of iso-octyl acrylate/N-vinyl pyrrolidone in ethyl acetate solvent at a coating weight of 30-40 g/m$^2$ dry weight using a conventional bar coater, slit into rolls having pinked edges and qualitatively evaluated for conformability as a medical tape. All three tapes were judged to have adequate hand or conformability but all three were also judged to have inferior MVT properties.

EXAMPLES 5-21

Film samples were prepared according to the procedures of Examples 1-4 except that a variety of additives designed to improve the MVT properties of the film were incorporated into the polymeric resin blend prior to extrusion. In all cases, the additive and 2.5 % by weight TiO$_2$ pigment/polypropylene blend was first custom master batched with the PE/EVA copolymer and 1% by weight ethylene glycol by C.B. Edwards Co., Minneapolis, MN, before blending with the polypropylene. All of the films exhibited good machine direction and cross-direction tear properties and were tested for their moisture vapor transmission properties (reported in Table II).

## TABLE II

| Example No. | Additive | PP & Pigment (pph) | PE/EVA (pph) | Additive (pph) | MVT $(g/m_2/24$ hr) |
|---|---|---|---|---|---|
| 5 | Control | 100 | - | - | 13.2 |
| 6 | Beads[1] | 80 | 10 | 10 | 48.3 |
| 7 | Airvol[2] | 89 | 10 | 1 | 92.7 |
| 8 | Airvol[2] | 85 | 10 | 5 | 98.7 |
| 9 | Airvol[2] | 80 | 10 | 10 | 105.3 |
| 10 | Methocel[3] | 89 | 10 | 1 | 32.9 |
| 11 | Methocel[3] | 85 | 10 | 5 | 36.3 |
| 12 | Methocel[3] | 80 | 10 | 10 | Holes |
| 13 | PNVP[4] | 89 | 10 | 1 | <10 |
| 14 | PNVP[4] | 85 | 10 | 5 | 65.8 |
| 15 | PNVP[4] | 80 | 10 | 10 | 177.8 |
| 16 | Carbopol[5] | 89 | 10 | 1 | <10 |
| 17 | Carbopol[5] | 85 | 10 | 5 | 32.9 |
| 18 | Carbopol[5] | 80 | 10 | 10 | 32.8 |
| 19 | Cab-O-Sil[6] | 89 | 10 | 1 | 72.4 |
| 20 | Cab-O-Sil[6] | 85 | 10 | 5 | 72.4 |
| 21 | Cab-O-Sil[6] | 80 | 10 | 10 | 52.7 |

1. E-22 Soda Lime Borosilicate glass beads, 35 micron average diameter, density 0.22 g/cc, available from 3M, St. Paul, MN.

2. Airvol[TM] Polyvinyl Alcohol 523 (87-89% hydrolyzed, medium viscosity), available from Air Products, Allentown, PA.

3. Methocel[TM] methylcellulose, hydroxypropyl methyl cellulose , available from Dow Chemical, Midland, MI.

4. Poly(N-Vinyl Pyrrolidone) #86645, 10,000 MW, available from Aldrich Chemical, Milwaukee, WI.

5. Carbopol[TM] 934P , an Acrylic Acid Homopolymer, available from B. F. Goodrich, Cleveland, Ohio.

6. Cab-O-Sil[TM] M-5 Fumed Silica powder, available from Cabot Corp., Reading, PA.

EXAMPLES 22-28

Film samples were prepared according to the procedures of Examples 5-21 except that the casting roll had a truncated pyramid pattern set at 45° relative to the length of the casting roll to produce a diamond pattern on the extruded film. The pyramid projections had a 1.02 mm (40 mil) base, a 0.25 mm (10 mil) truncation and a base to truncation distance of 0.25 mm (10 mil). The pyramid bases were spaced 1.02 mm (40 mil) from adjacent pyramids with the spacing land having a concave profile relative to the pyramids, the deepest point of the curve being 0.125 mm (5 mil) below the base of the pyramids. Nominal film thickness was about 12.5 mm (5 mil), with the thinnest sections of the film having a nominal thickness of about 0.25 - 0.51 mm (1 - 2 mil). All film samples exhibited good machine direction and cross-direction tear properties. Composition of the film samples along with MVT and tensile properties are reported in Table III.

**TABLE III**

| Example | 22 | 23 | 24 | 25 | 26 | 27 | 28 |
|---|---|---|---|---|---|---|---|
| PP + Pigment (pph) | 100 | 90 | 87.7 | 87.7 | 87.7 | 87.7 | 87.7 |
| PE/EVA (pph) | 0 | 10 | 10 | 10 | 10 | 10 | 10 |
| Additive (pph) | 0 | 0 | 2.3 | 2.3 | 2.3 | 2.3 | 2.3 |
| Additive | – | – | PVA[1] | PNVP[2] | Bead[3] | Bead[4] | Aspen[5] |
| MD Tensile (kg/cm width) | 2.41 | 2.43 | 2.14 | 2.34 | 2.45 | 2.10 | 2.68 |
| Hand (g total) | 176 | 160 | – | 167 | – | 169 | 171 |
| MVT (g/m$^2$/24 hrs.) | <20 | <20 | – | 50 | – | 35 | 100 |

1. Airvol™ Polyvinyl Alcohol 523 (87-89% hydrolyzed, medium viscosity), available from Air Products, Allentown, PA.

2. Poly(N-Vinyl Pyrrolidone) #86645, 10,000 MW, available from Aldrich Chemical, Milwaukee, WI.

3. E-22 Soda Lime Borosilicate glass beads, 35 micron average diameter, density 0.22 g/cc, available from 3M, St. Paul, MN.

4. S-60 Soda Lime Borosilicate glass beads, 40 micron average diameter, density 0.60 g/cc, available from 3M, St. Paul, MN.

5. Aspen™ Resin #6806, a Hydrophilic Polyolefin Resin, available from Dow Chemical, Midland MI.

The film samples from Examples 22-28 were then coated with an acrylate based pressure-sensitive adhesive similar to that described in U.S. Pat. No. RE24,906 (Ulrich) that comprised a 91/9 copolymer of iso-octyl acrylate/N-vinyl pyrrolidone in ethyl acetate solvent or a phenolic cured rubber-based pressure-sensitive adhesive similar to that described in U.S. Pat. No. 2,708,192 (Joesting et. al.) (comprising 100 parts smoke-sheet rubber, 60 parts tackifying resin, 20 parts phenolic resin, 20 parts zinc oxide and 20 parts titanium dioxide) in heptane at a dry coating weight of about 36-38 g/m$^2$ using a conventional bar coater. MVT data for the pressure-sensitive adhesive tape constructions are reported in Table IV. In view of the significant increase in MVT properties of some of the tape constructions relative to the uncoated films, solvent (ethyl acetate) was applied to each film sample and the film dried in an oven at about 104°C (220°F) for two minutes to determine if exposure to the solvent would increase the MVT properties of the film. MVT data for the solvent treated films is reported in Table IV.

TABLE IV

| Film Example | MVT (g/M$^2$/24 hrs) | | |
|---|---|---|---|
| | Acrylic PSA | Rubber PSA | Treated Film |
| 22 | <20 | <20 | <20 |
| 23 | 50 | 40 | 80 |
| 24 | 1250 | 1160 | <20 |
| 25 | 1330 | 1500 | 65 |
| 26 | 525 | 1870 | 65 |
| 27 | 420 | 1715 | 260 |
| 28 | 605 | 895 | <20 |

Data in Table IV shows that treating the backings of the present invention with a solvent increases the MVT properties of the film, and that coating the backings of the present invention with a solvent-based adhesive significantly increases the MVT properties of the film.

Claims

1. An adhesive composite comprising in combination a predominantly crystalline isotactic polypropylene film backing prepared from a resin comprising by weight
   a) 65 to 98 percent of isotactic polypropylene,

b) 1 to 20 percent of a polyethylene/ethyl vinyl acetate copolymer comprising 5 to 40 percent by weight of ethyl vinyl acetate, and

c) 1 to 15 percent of a moisture vapor transmission rate enhancing material,

and a layer of normally tacky, pressure-sensitive adhesive, said backing having an embossed pattern on at least one side that provides for varied thicknesses of the backing such that the backing has an overall thickness of 0.075 to 0.115 mm. with regions of the backing having a thickness of less than one-half the overall thickness of the backing.

2. An adhesive composite of claim 1, wherein said polyethylene/ethyl vinyl acetate copolymer comprises 25 to 35 percent by weight of ethyl vinyl acetate.

3. An adhesive composite of claim 1, wherein said film backing comprises by weight

a) 78 to 86 percent of isotactic polypropylene,

b) 8 to 12 percent of a polyethylene/ethyl vinyl acetate copolymer comprising 25 to 35 percent by weight of ethyl vinyl acetate, and

c) 8 to 12 percent of a moisture vapor transmission rate enhancing material.

4. An adhesive composite of claim 1, wherein said moisture vapor transmission rate enhancing material is selected from the group consisting of glass beads, silica powder, polyvinyl alcohol, poly(N-vinyl pyrrolidone), acrylic acid homopolymer or cellulosic fibers.

5. An adhesive composite of claim 1, wherein said moisture vapor transmission rate enhancing material is a polyvinyl alcohol.

6. An adhesive composite of claim 1, wherein said moisture vapor transmission rate enhancing material is poly(N-vinyl pyrrolidone).

7. An adhesive composite of claim 1, wherein said film backing has been exposed to an organic solvent before application of the adhesive.

8. An adhesive composite of claim 1, wherein said adhesive is cast from a solvent.

9. An adhesive composite of claim 1, wherein said embossed pattern is a pattern of truncated pyramids.

10. An adhesive composite of claim 1, wherein 20% of the film backing has a thickness of no more than 0.05 mm.

11. An adhesive composite of claim 1, wherein 20% of the backing has a thickness of no more than 0.03 mm.

12. An adhesive composite of claim 1, which is an elongate strip of normally tacky, pressure-sensitive adhesive tape wound convolutely upon itself about a core to form a roll.

**Patentansprüche**

1. Klebverbundstoff, umfassend in Kombination einen überwiegend kristallinen isotaktischen Polypropylen-Filmträger, hergestellt aus einem Kunstharz, umfassend:

(a) 65 % ... 98 Gewichtsprozent isotaktisches Polypropylen,

(b) 1 % ... 20 Gewichtsprozent eines Polyethylen/Ethylvinylacetat-Copolymers mit 5 % ... 40 Gewichtsprozent Ethylvinylacetat und

(c) 1 % ... 15 Gewichtsprozent eines Materials zum Erhöhen der Wasserdampfdurchlässigkeit,

sowie eine Schicht von normalklebrigem Haftklebstoff, wobei der Träger auf mindestens einer Seite ein geprägtes Muster aufweist, das in einer solchen Weise für unterschiedliche Dicken des Trägers sorgt, daß der Träger eine Gesamtdicke von 0,075 ... 0,115 mm mit Bereichen des Trägers aufweist, die eine Dicke von weniger als die Hälfte der Gesamtdicke des Trägers haben.

2. Klebverbundstoff nach Anspruch 1, bei welchem das Polyethylen/Ethylvinylacetat-Copolymer 25 % ... 35 Gewichtsprozent Ethylvinylacetat aufweist.

**3.** Klebverbundstoff nach Anspruch 1, bei welchem der Filmträger aufweist:
(a) 78 % ... 86 Gewichtsprozent isotaktisches Polypropylen,
(b) 8 % ... 12 Gewichtsprozent eines Polyethylen/Ethylvinylacetat-Copolymers mit 25 % ... 35 Gewichtsprozent Ethylvinylacetat und
(c) 8 % ... 12 Gewichtsprozent eines Materials zum Erhöhen der wasserdampfdurchlässigkeit.

**4.** Klebverbundstoff nach Anspruch 1, bei welchem das Material zum Erhöhen der Wasserdampfdurchlässigkeit ausgewählt wird aus der Gruppe, bestehend aus: Glaskugeln, Silicapulver, Polyvinylalkohol, Poly(N-vinylpyrrolidon), Acrylsäure-Homopolymer oder Cellulosefasern.

**5.** Klebverbundstoff nach Anspruch 1, bei welchem das Material zum Erhöhen der Wasserdampfdurchlässigkeit ein Polyvinylalkohol ist.

**6.** Klebverbundstoff nach Anspruch 1, bei welchem das Material zum Erhöhen der Wasserdampfdurchlässigkeit Poly(N-vinylpyrrolidon) ist.

**7.** Klebverbundstoff nach Anspruch 1, bei welchem der Filmträger vor dem Auftragen des Klebstoffes einem organischen Lösemittel ausgesetzt wurde.

**8.** Klebverbundstoff nach Anspruch 1, bei welchem der Klebstoff aus einem Lösemittel aufgegossen wird.

**9.** Klebverbundstoff nach Anspruch 1, bei welchem das geprägte Muster ein Muster aus Pyramidenstümpfen ist.

**10.** Klebverbundstoff nach Anspruch 1, bei welchem 20 Prozent des Filmträgers eine Dicke von nicht mehr als 0,05 mm aufweisen.

**11.** Klebverbundstoff nach Anspruch 1, bei welchem 20 Prozent des Filmträgers eine Dicke von nicht mehr als 0,03 mm aufweisen.

**12.** Klebverbundstoff nach Anspruch 1, der ein langgestreckter Streifen eines normalklebrigen Haftklebebandes ist und zur Bildung einer Rolle um eine Spule aufgewickelt ist

## Revendications

**1.** Composite adhésif comprenant en combinaison un support pelliculaire en polypropylène isotactique, façon prédominante cristallin et préparé à partir d'une résine comprenant en poids
a) 65 à 98 % de polypropylène isotactique,
b) 1 à 20 % d'un copolymère polyéthylène-acétate de vinyle/éthyle comprenant 5 à 40 % en poids d'acétate de vinyle/éthyle, et
c) 1 à 15 % d'un matériau améliorant le taux de transmission de la vapeur d'eau,
et une couche d'un adhésif normalement poisseux, sensible à la pression, ledit support ayant sur au moins un côté, un motif embossé qui fournit des épaisseurs variables pour ledit support de telle façon que ledit support ait une épaisseur totale de 0,075 à 0,115 mm avec des régions où l'épaisseur est inférieure à la moitié de l'épaisseur totale.

**2.** Composite adhésif suivant la revendication 1, dans lequel ledit copolymère polyéthylène-acétate de vinyle/éthyle comprend 25 à 35 % en poids d'acétate de vinyle/éthyle.

**3.** Composite adhésif suivant la revendication 1, dans lequel ledit support comprend en poids
a) 78 à 86 % de polypropylène isotactique,
b) 8 à 12 % d'un copolymère polyéthylène-acétate de vinyle/éthyle comprenant 25 à 35 % en poids d'acétate de vinyle/éthyle, et
c) 8 à 12 % d'un matériau améliorant le taux de transmission de la vapeur d'eau.

**4.** Composite adhésif suivant la revendication 1, dans lequel ledit matériau améliorant le taux de transmission de la vapeur d'eau est choisi parmi l'ensemble constitué par des billes de verre, une poudre de silice, un alcool polyvinylique, une poly(N-vinylpyrrolidone), un homopolymère de l'acide acrylique ou des fibes cellulosiques.

5. Composite adhésif suivant la revendication 1, dans lequel ledit matériau améliorant le taux de transmission de la vapeur d'eau est un alcool polyvinylique.

6. Composite adhésif suivant la revendication 1, dans lequel ledit matériau améliorant le taux de transmission de la vapeur d'eau est une poly(N-vinylpyrrolidone).

7. Composite adhésif suivant la revendication 1, dans lequel ledit support pelliculaire a été exposé à un solvant organique avant l'application de l'adhésif.

8. Composite adhésif suivant la revendication 1, dans lequel ledit adhésif est déposé au moyen d'un solvant.

9. Composite adhésif suivant la revendication 1, dans lequel ledit motif embossé est un motif de pyramides tronquées.

10. Composite adhésif suivant la revendication 1, dans lequel 20 % du support pelliculaire a une épaisseur non supérieure à 0,05 mm.

11. Composite adhésif suivant la revendication 1, dans lequel 20 % du support pelliculaire a une épaisseur non supérieure à 0,03 mm.

12. Composite adhésif suivant la revendication 1, qui est un ruban allongé de bande adhésive normalement poisseuse, sensible à la pression, et roulée sur elle-même autour d'une âme pour former un rouleau.

FIG.1

FIG.2

FIG.3

FIG. 4

FIG. 5

FIG. 6

FIG. 7